# EUROPEAN PATENT APPLICATION

(11) **EP 2 170 022 A1**
(43) Date of publication of application: **31.03.2010**
(21) Application number: 08016930.3
(22) Date of filing: 25.09.2008
(51) Int. Cl.: H05H 1/24

(54) **Plasma applicator and corresponding method**

(71) Applicant: Max-Planck-Gesellschaft zur Förderung der Wissenschaften e.V., 80539 München (DE)
(72) Inventor: Morfill, Gregor, 81927 München (DE); Steffes, Bernd, 85748 Garching (DE); Shimizu, Tetsuji, 85748 Garching (DE); Pompl, René, 80798 München (DE); Nosenko, Tetiana, 85748 Garching (DE); Stolz, Wilhelm, 80638 München (DE); Isbary, Georg, 80796 München (DE); Schmidt, Hans-Ulrich, 82049 Pullach (DE)
(74) Representative: Beier, Ralph

(57) **Abstract**

The invention relates to a plasma applicator (1) for applying a non-thermal plasma to a surface (2), particularly for the plasma treatment of living tissue and especially for the plasma treatment of wounds (2), comprising a sealing cover (4) for covering a portion of the surface thereby enclosing a cavity between the sealing cover (4) and the surface (2), wherein the non-thermal plasma is provided in the cavity so that the non-thermal plasma contacts the surface (2). Further, the invention relates to a corresponding method.

## Description

### Field of the invention

The invention relates to a plasma applicator for applying a non-thermal plasma to a surface, particularly for the plasma treatment of living tissue and especially for the treatment of wounds.

Further, the invention relates to a corresponding method for applying a non-thermal plasma to a locally bounded surface, particularly for the treatment of wounds.

### Background of the invention

The use of non-thermal plasmas for the treatment of wounds and especially for the in-vivo sterilisation, decontamination or disinfection of wounds is disclosed, for example, in WO 2007/031250 A1 and PCT/EP2008/003568.

However, it is desirable to improve the healing effect of the wound treatment with the non-thermal plasma.

### Summary of the invention

Therefore, it is a general object of the invention to improve the sterilizing effect of the plasma and the wound healing in a plasma therapy.

This object is achieved by a novel plasma applicator and a corresponding method according to the independent claims.

The plasma applicator according to the invention comprises a sealing cover (e.g. an adhesive plaster) for covering a portion of the surface thereby enclosing a cavity between the sealing cover and the surface, wherein a non-thermal plasma is provided in the cavity so that the non-thermal plasma in the cavity contacts the surface thereby sterilizing the surface and improving the wound healing.

The non-thermal plasma is preferably generated within the cavity by an electrode arrangement and/or an antenna arrangement which produces the non-thermal plasma.

However, it is alternatively possible that the plasma is generated outside the cavity and then introduced into the cavity through a conduit.

In one embodiment of the invention, the electrode or antenna arrangement of the plasma applicator comprises a single electrode only, so that the treated surface forms a counter electrode. In this embodiment, the treated object (e.g. a patient) is preferably electrically grounded and high-voltage is applied to the single electrode thereby producing the plasma in the cavity.

In another embodiment of the invention, the electrode or antenna arrangement of the plasma applicator comprises at least two separate electrodes for a bipolar generation of the electrode or antenna arrangement. In this embodiment, the generation of the plasma takes place between the separate electrodes so that it is not necessary to electrically ground the patient.

In a preferred embodiment of the invention, the electrode or antenna arrangement is spiral-shaped, particularly in the form of an Archimedien spiral having a constant separation distance between successive turnings of a spiral. However, the electrode or antenna arrangement may be in the form of any other type of spiral, e.g. a logarithmic spiral. Further, it is alternatively possible that the electrode or antenna arrangement is mesh-shaped. However, it should be noted that the invention is not restricted to the afore-mentioned exemplary forms of electrode or antenna arrangements.

The electrode arrangement is preferably flexible and can be adapted to the wound geometry. For example, the size of the electrode geometry can be adapted to the size of the wound so that the entire wound is covered by the plasma applicator. Therefore, the afore-mentioned electrode arrangement can be cut or tailored to the wound geometry. Further, the shape of the plasma applicator can be adapted to the shape of the wound so that the plasma applicator follows the contour of the wound.

In the preferred embodiment, the plasma applicator according to the invention further comprises a gas-permeable padding which is arranged within the cavity. The padding is preferably porous, e.g. in the form of a sponge, an aerogel or spheres of a polymer. Alternatively, the padding may consist of sacks filled with sand, quartz or the like.

Further, the padding is preferably substantially non-compressible and/or permeable to gas, preferably in the pressure range down to approximately 10hPa.

Moreover, the padding can be functionalised by coating or impregnating the padding with a substance which is improving the plasma generation and/or which has a medical effect, particularly a sterilizing effect. For example, the padding can be coated or impregnated with a bactericide, a fungicide and/or an antiviral substance.

It should further be noted that the padding is preferably flexible so that it is adaptable to the contour of the treated surface.

Further, the electrode or antenna arrangement is preferably permeable to gas so that the electrode or antenna arrangement does not form a barrier for the carrier gas/plasma within the cavity.

In one alternative embodiment of the invention, the electrode or antenna arrangement is integrated or embedded into the padding so that the padding defines the relative position of the electrode or antenna arrangement.

In another embodiment of the invention, the electrode or antenna arrangement is located above the padding between the padding and the sealing cover and/or attached to the inner surface of the sealing cover.

It is also possible to functionalise the electrode or antenna arrangement by coating or impregnating with a substance which is improving the plasma generation and/or which has a medical effect, particularly a sterilizing effect. Therefore, the electrode or antenna arrangement can be coated or impregnated with a bactericide, a fungicide and/or an antiviral substance.

It should further be noted that the electrode or antenna arrangement is preferably substantially two-dimensional so that the electrode or antenna arrangement forms a mat. For example, the electrode or antenna arrangement can be a perforated foil. Further, the electrode or antenna arrangement is preferably flexible so that the electrode or antenna arrangement can be adapted to the wound geometry.

Moreover, the electrode or antenna arrangement is preferably flexible so that it is adaptable to the contour of the treated surface.

Further, the sealing cover is preferably substantially impermeable to gas. This is preferred since it allows to create a plasma environment surrounding the treated surface.

Moreover, the sealing cover is preferably at least partially adhesive for adhering the plasma applicator to the surface. Particularly, the plasma applicator according to the invention preferably comprises an adhesive boarder strip which can be adhered to the skin of a patient surrounding a wound.

Further, it is also possible to functionalise the sealing cover by coating or impregnating the sealing cover with a substance which is improving the plasma generation and/or which has a medical effect, particularly a sterilizing effect. Therefore, the sealing cover can be coated or impregnated with a bactericide, a fungicide and/or an antiviral substance.

It should further be noted that the sealing cover is preferably flexible so that it is adaptable to the contour of the treated surface. This is important when the plasma applicator is attached to a curved surface of the skin of a patient. Therefore, the entire plasma applicator is preferably flexible.

It should further be noted that the plasma applicator according to the invention preferably comprises a gas inlet for introducing a carrier gas into the cavity between the sealing cover and the treated surface, wherein the gas inlet can be connected to a gas source. The gas source can provide a carrier gas (e.g. argon, ambient air). Alternatively, the gas source can provide a mixture of the carrier source and any additive which is improving the wound healing and/or which is improving the plasma characteristics and/or the sterilizing effect. Alternatively, the plasma applicator according to the invention may comprise several gas inlets for introducing the carrier gas (e.g. ambient air, argon) and the additive (e.g. silver compounds) separately.

Further, the plasma applicator according to the invention preferably comprises a gas outlet for exhausting gas out of the cavity, wherein the gas outlet can be connected to a suction pump. The gas outlet allows a reduction of the pressure in the cavity before the carrier gas (e.g. argon) is introduced into the cavity through the gas inlet. Thus, it is easy to replace the ambient air in the cavity by the carrier gas. Further, a reduction of the pressure in the cavity facilitates the plasma generation in the cavity.

However, the reduction of the pressure in the cavity can result in a compression of the flexible padding due to the atmospheric pressure on the outside of the plasma applicator. Therefore, a rigid strut (e.g. a box frame or a box base) can be arranged in the cavity thereby holding up the flexible padding even in case of a low pressure in the cavity.

Moreover, the plasma applicator according to the invention preferably comprises an electrical contact being connected with the electrode or antenna arrangement for generating the plasma, wherein the electrical contact can be connected to an external high-voltage source for exciting the electrode or antenna arrangement thereby producing the plasma in the cavity.

It is already apparent from the above description that the plasma applicator according to the invention is preferably designed as an adhesive plaster which can be adhered to the skin in order to apply the non-thermal plasma to the skin.

The invention further encompasses a plasma application device comprising the afore-mentioned plasma applicator according to the invention, preferably in the form of an adhesive plaster.

Further, the plasma application device according to the invention preferably comprises a high-voltage source, which is connected to the electrical contact of the plasma applicator for energizing the electrode or antenna arrangement of the plasma applicator.

Further, the plasma application device according to the invention preferably comprises a suction pump being connected to the gas outlet of the plasma applicator for drawing gas out of the cavity of the plasma applicator thereby reducing the pressure in the cavity.

Moreover, the plasma application device according to the invention preferably comprises a gas source being connected to the gas inlet of the plasma applicator for introducing a carrier gas into the cavity, wherein the carrier gas can be the ambient air, argon, or a mixture of several gases with additives, which are improving the wound healing and/or the plasma generation.

The gas flow into the cavity of the plasma applicator is preferably controlled by a flow controller and/or an inlet valve which is arranged between the gas source and the gas inlet of the plasma applicator.

Further, the gas flow out of the cavity is preferably controlled by an outlet valve which is arranged between the gas outlet of the plasma applicator and the suction pump.

Moreover, the plasma application device according to the invention preferably comprises a control unit for controlling the inlet valve, the outlet valve and/or the high-voltage generator.

Further, the invention encompasses an operating method for the afore-mentioned plasma applicator.

For example, the plasma applicator can operate continuously for a specific treatment time so that the plasma applicator is switched on at the beginning of the treatment and switched off at the end of the treatment. The treatment time can be adjusted according to medical requirements.

Alternatively, a pulsed operation of the plasma applicator is possible, wherein the plasma applicator is operating with a specific pulse rate so that the plasma applicator is continuously switched on and off during the treatment. The pulse rate and the treatment time can be adjusted according to medical requirements.

Further, the invention also encompasses the novel use of the afore-mentioned plasma applicator for the treatment of wounds, living tissue or skin diseases or skin disorders.

The invention further encompasses a novel method for applying a non-thermal plasma to a locally bounded surface, particularly for the treatment of wounds.

The method according to the invention comprises the step of attaching a sealing cover to the locally bounded surface thereby providing a cavity between the sealing cover and the locally bounded surface.

Further, the method according to the invention comprises the step of providing a non-thermal plasma within the cavity in contact with the locally bounded surface, so that the non-thermal plasma improves the wound healing.

Moreover, the method according to the invention preferably comprises the step of sealing the cavity substantially gastight so that the pressure in the cavity can be reduced by exhausting gas out of the cavity. Therefore, the method according to the invention preferably comprises the step of reducing the pressure within the cavity by exhausting gas out of the cavity.

Further, the method according to the invention preferably comprises the step of introducing a carrier gas into the cavity and finally the step of exciting an electrode or antenna arrangement in the cavity thereby generating the non-thermal plasma in the cavity.

Therefore, the plasma is preferably generated *in situ,* i.e. within the cavity. However, it is alternatively possible that the plasma is generated in a separate plasma generator and then introduced into the cavity through a conduit.

In case of a single electrode or antenna arrangement, the method according to the invention preferably comprises the steps of electrically grounding the treated surface and exciting the electrode after the grounding of the treated surface.

It should further be noted that the non-thermal plasma according to the invention preferably comprises a gas temperature (i.e. the temperature of the atoms and molecules) below +40°C, when measured on the treated surface.

Further, the pressure of the plasma within the plasma applicator is preferably in the range of 1hPa-1.200hPa and more preferably in the range of 10hPa-500hPa, wherein a pressure of approximately 100hPa is preferred.

Moreover, the degree of ionization (i.e. the percentage of the ionized atoms or molecules) of the carrier gas is preferably above 1·1⁻⁹, 2·10⁻⁹, 5·10⁻⁹, 10⁻⁸, 2·10⁻⁸ or 5·10⁻⁸.

The invention and its particular features and advantages will become apparent from the following detailed description considered with reference to the accompanying drawings.

### Brief description of the drawings

- Figure 1A: is a cross-sectional view of a plasma applicator according to the invention along line A-A in Figure 1B,
- Figure 1B: is a top view of the plasma applicator according to Figure 1A on the skin of a patient,
- Figure 2: is a cross-sectional view of a plasma applicator according to another embodiment of the invention.
- Figure 3: is a flowchart illustrating the method according to the invention for applying a non-thermal plasma to a surface.
- Figure 4A: is a schematic view of an electrode arrangement in the form of an Archimedian spiral.
- Figure 4B: is another embodiment of an electrode arrangement which can be used in the afore-mentioned plasma applicator according to the invention,
- Figure 5: is a cross-sectional view of a plasma applicator according to another embodiment of the invention.

### Detailed description of the drawings

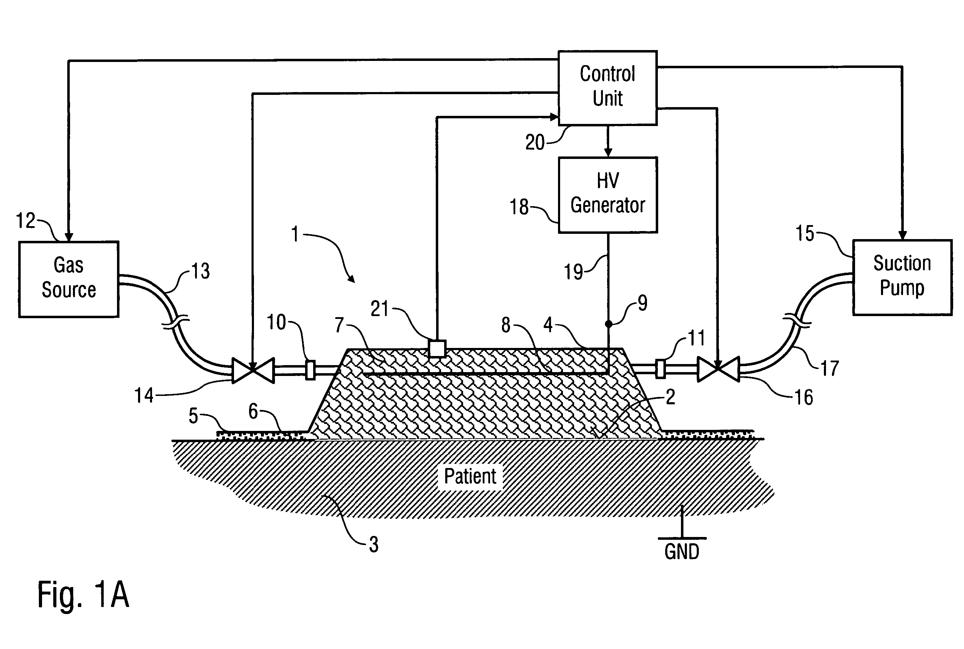

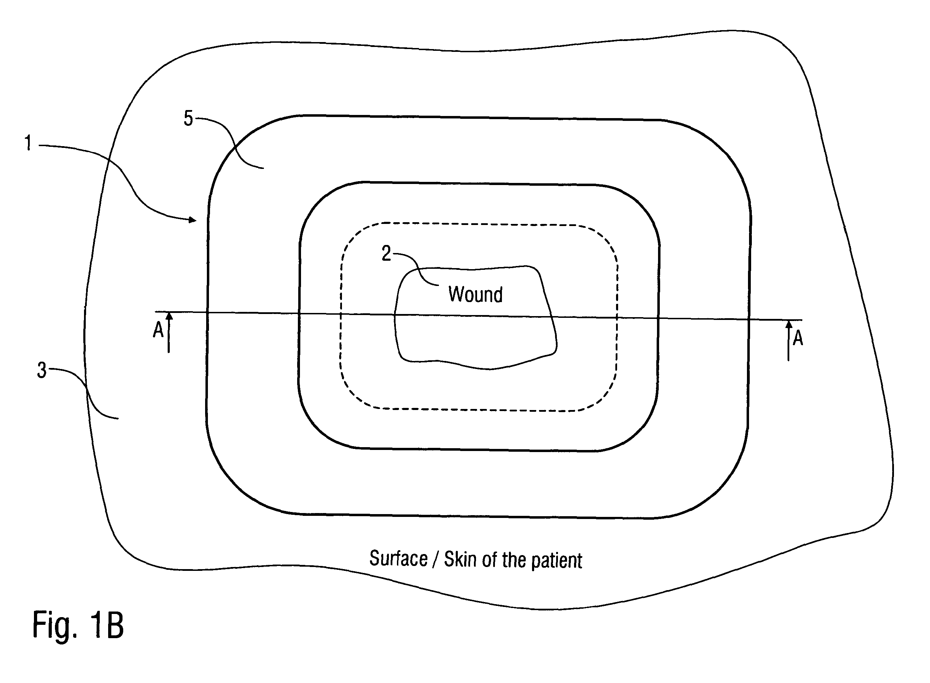

Figures 1A and 1B illustrate a preferred embodiment of a plasma applicator 1 for generating and applying a non-thermal plasma to a locally bounded wound 2 of a patient 3.

The plasma applicator comprises a flexible and gas tight sealing cover 4 which can be adhered to the skin surrounding the wound 2. Therefore, the sealing cover 4 comprises an adhesive boarder strip 5 which is coated with an adhesive 6 for adhering the boarder strip 5 of the sealing cover 4 to the skin of the patient 3 surrounding the wound 2.

The sealing cover 4 encloses a cavity between the wound 2 and the sealing cover 4, wherein the cavity is filled with a gas-permeable and porous padding 7 which is functionalised by impregnating the padding 7 with a substance which is improving the wound healing.

Further, the plasma applicator 1 comprises a single electrode 8 for producing the non-thermal plasma in the cavity between the sealing cover 4 and the wound 2. The electrode 8 is connected to an external electrical contact 9 through a gastight feedthrough.

Further, the plasma applicator 1 comprises a gas inlet 10 for introducing a carrier gas into the cavity between the sealing cover 4 and the wound 2.

Moreover, the plasma applicator 1 comprises a gas outlet 11 for exhausting gas out of the cavity between the sealing cover 4 and the wound 2.

The gas inlet 10 of the plasma applicator 1 is connected to a gas source 12 via a conduit 13 and an inlet valve 14.

Further, the gas outlet 11 is connected to a suction pump 15 via an outlet valve 16 and a conduit 17.

Moreover, the electrical contact 9 of the plasma applicator 1 is connected to a high-voltage generator 18 through a cable 19.

Finally, the plasma application device shown in Figures 1A and 1B comprises a control unit 20 which is controlling the gas source 12, the suction pump 15, the high-voltage generator 18, the inlet valve 14 and the outlet valve 16.

Further, the control unit 20 is connected to a pressure sensor 21 which measures the pressure in the plasma applicator 1. The control unit 20 controls the inlet valve 14 and the outlet valve 16 in such a way that a target value of about P_{TARGET}=100hPa is adjusted.

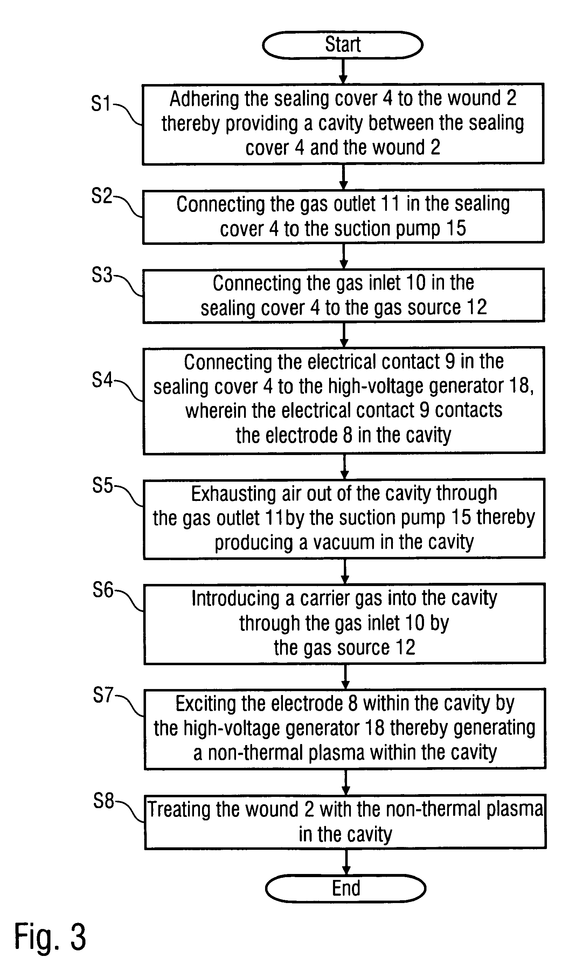

In the following, the operation of the afore-mentioned plasma application device is illustrated with reference to the flow chart shown in Figure 3.

In a first step S1, the plasma applicator 1 is adhered to the skin of the patient 3 surrounding the wound 2.

In a second step S2, the gas outlet 11 of the plasma applicator 1 is connected to the suction pump 15.

Then, the gas inlet 10 of the plasma applicator 1 is connected to the gas source 12 in step S3.

In another step S4, the high-voltage generator 18 is connected to the electrical contact 9 of the plasma applicator 1.

Then, in a step S5, the control unit 20 closes the inlet valve 14 and opens the outlet valve 16 so that the suction pump 15 draws air out of the plasma applicator 1 thereby reducing the pressure in the cavity between the sealing cover 4 and the skin of the patient 3.

In a next step S6, the control unit 20 closes the outlet valve 16 and switches the suction pump 15 off. Further, the control unit 20 opens the inlet valve 14 so that the gas source 12 delivers a carrier gas (e.g. argon) into the plasma applicator 1.

Then, the control unit 20 closes the inlet valve 14 and activates the high-voltage generator 18 in a step S7, so that a non-thermal plasma is produced between the single electrode 8 and the electrically grounded patient 3.

Then, in step S8, the patient 3 is treated with the non-thermal plasma.

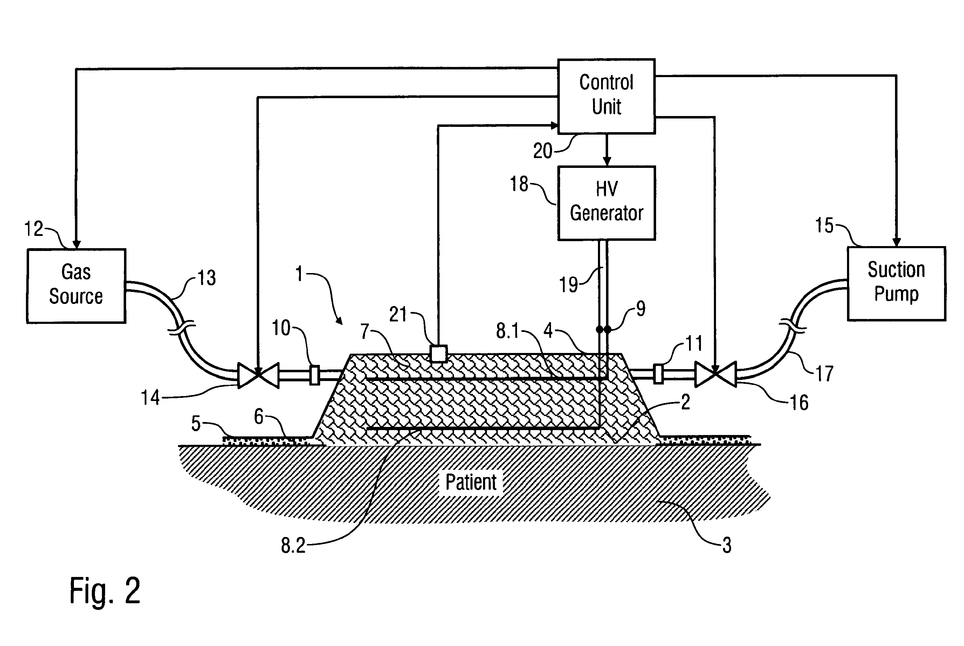

Figure 2 shows a cross-sectional view of another embodiment of a plasma applicator 1 according to the invention which is similar to the embodiment shown in Figure 1A and 1B. Therefore, reference is made to the above description and the same reference numerals are used for corresponding details, parts and components.

One characteristic of this embodiment is that the electrode arrangement for producing the plasma in the plasma applicator 1 comprises two separate electrodes 8.1, 8.2 for a bipolar generation of the plasma between the electrodes 8.1, 8.2. This is advantageous since it is not necessary to electrically ground the patient 3.

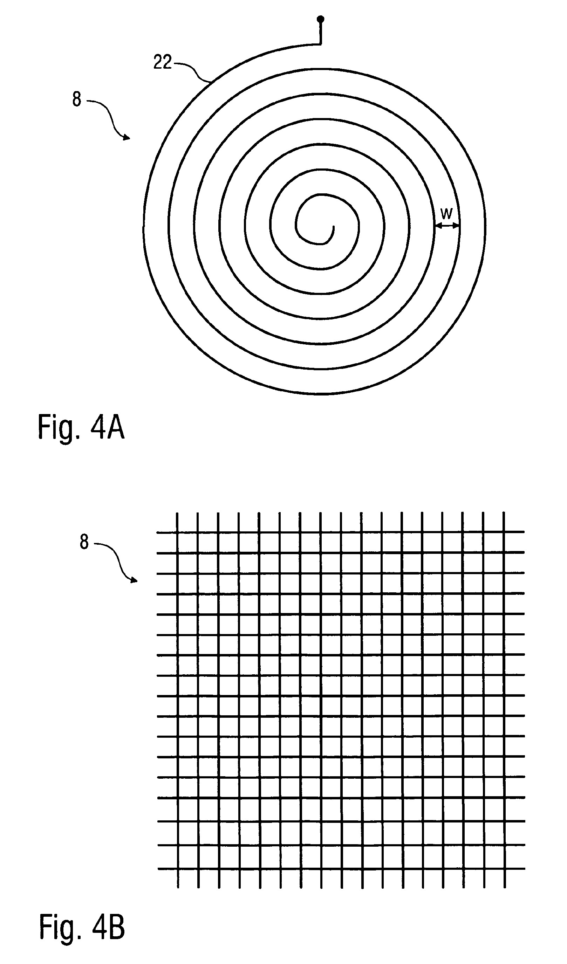

Figure 4a shows an exemplary embodiment of the electrode 8 in the form of an Archimedian spiral with a constant distance w between successive turnings 22 of the spiral.

Figure 4B shows another embodiment of the electrode 8 in the form of a mesh.

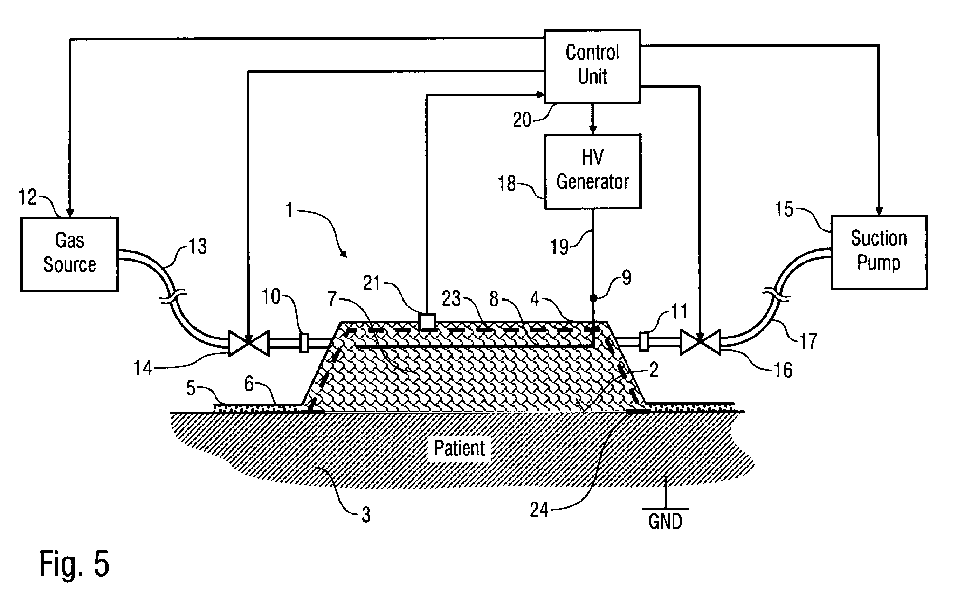

Figure 5 shows a cross-sectional view of another embodiment of a plasma applicator 1 according to the invention which is similar to the embodiment shown in Figure 1A and 1B. Therefore, reference is made to the above description and the same reference numerals are used for corresponding details, parts and components.

One distinctive feature of this embodiment is that there is a rigid strut 23 arranged in the cavity thereby preventing the compression of the padding 7 in case of a low pressure in the cavity.

The strut 23 is gas permeable so that the pressure sensor 21 can measure the gas pressure within the cavity although the pressure sensor 21 is arranged outside the strut 23.

Further, the strut 23 comprises a circumferential base 24 resting on the skin of the patient 3 outside the wound 2 so that the wound 2 is not affected by the pressure exerted by the base 24 and plasma applicator 1 dos not cause any pain to the patient 3.

Although the invention has been described with reference to the particular arrangement of parts, features and the like, these are not intended to exhaust all possible arrangements of features, and indeed many other modifications and variations will be ascertainable to those of skill in the art.

### List of reference numerals

- 1: Plasma applicator
- 2: Wound
- 3: Patient
- 4: Sealing cover
- 5: Border strip of the sealing cover
- 6: Adhesive
- 7: Padding
- 8: Electrode
- 8.1: Electrode
- 8.2: Electrode
- 9: Electrical contact
- 10: Gas inlet
- 11: Gas outlet
- 12: Gas source
- 13: Conduit
- 14: Inlet valve
- 15: Suction pump
- 16: Outlet valve
- 17: Conduit
- 18: High-voltage generator
- 19: Cable
- 20: Control unit
- 21: Pressure sensor
- 22: Turning of spiral
- 23: Strut
- 24: Base of strut

## Claims

1. Plasma applicator (1) for applying a non-thermal plasma to a surface (2), particularly for the plasma treatment of wounds (2), **characterized by** a sealing cover (4) for covering a portion of the surface thereby enclosing a cavity between the sealing cover (4) and the surface (2), wherein the non-thermal plasma is provided in the cavity so that the non-thermal plasma contacts the surface (2).

2. Plasma applicator (1) according to claim 1, further comprising an electrode arrangement (8; 8.1, 8.2) or an antenna arrangement for exciting the non-thermal plasma within the cavity.

3. Plasma applicator according to claim 2, wherein
a) the electrode arrangement (8) or the antenna arrangement comprises a single electrode only so that the treated surface (2, 3) forms a counter electrode, or
b) the electrode arrangement (8.1, 8.2) or the antenna arrangement comprises at least two separate electrodes for a bipolar excitation of the electrode arrangement (8.1, 8.2).

4. Plasma applicator (1) according to any of claims 2 or 3, wherein the electrode arrangement (8; 8.1, 8.2) is
a) spiral-shaped, particularly in the form of an Archimedien spiral, or
b) mesh-shaped.

5. Plasma applicator (1) according to any of the preceding claims, further comprising a gas permeable padding (7) being arranged within the cavity for contacting the surface (2).

6. Plasma applicator (1) according to claim 5, wherein the padding (7) is
a) porous, and/or
b) a sponge, and/or
c) substantially non-compressible, and/or
d) permeable to gas, and/or
e) functionalized by coating or impregnating with a substance which is improving the plasma generation and/or having a medical effect, particulary a sterilizing effect, and/or
f) flexible, so that it is adaptable to the contour of the treated surface (2), and/or
g) held up by a substantially rigid strut (23) which is arranged in the cavity thereby avoiding a compression of the padding (7) in case of a low pressure in the cavity.

7. Plasma applicator (1) according to any of claims 2 to 6, wherein the electrode arrangement (8; 8.1, 8.2) is
a) permeable to gas, and/or
b) integrated or embedded into the padding (7), and/or
c) arranged above the padding and/or between the padding (7) and the sealing cover (4), and/or
d) attached to the inner surface of the sealing cover (4), and/or
e) functionalized by coating or impregnating with a substance which is improving the plasma generation and/or having a medical effect, particulary a sterilizing effect, and/or
f) substantially two-dimensional, and/or
g) flexible so that it is adaptable to the contour of the treated surface (2), and/or
h) adapted to the size, shape and/or geometry of the wound (2) so that the plasma applicator (1) covers the entire wound (2).

8. Plasma applicator (1) according to any of the preceding claims, wherein the sealing cover (4) is
a) substantially impermeable to gas, and/or
b) at least partially adhesive for adhering the plasma applicator (1) to the surface, particularly in that it comprises an adhesive border strip (5) and/or
c) functionalized by coating or impregnating with a substance which is improving the plasma generation and/or having a medical effect, particulary a sterilizing effect, and/or
d) flexible, so that it is adaptable to the contour of the treated surface.

9. Plasma applicator (1) according to any of the preceding claims, further comprising:
a) a gas inlet (10) for introducing a carrier gas into the cavity, wherein the gas inlet (10) can be connected to a gas source (12), and/or
b) a gas outlet (11) for exhausting gas out of the cavity, wherein the gas outlet (11) can be connected to a suction pump (15), and/or
c) an electrical contact (9) being connected with the electrode arrangement (8; 8.1, 8.2), wherein the electrical contact (9) can be connected to a high-voltage source (18) for energizing the electrode arrangement (8; 8.1, 8.2) thereby exciting the plasma.

10. Plasma applicator (1) according to any of the preceding claims, wherein it is designed as an adhesive plaster which can be adhered to the skin.

11. Plasma application device comprising
a) a plasma applicator (1) according to any of the preceding claims, and/or
b) a high-voltage source (18) being connected to the electrical contact (9) of the plasma applicator (1), and/or
c) a suction pump (15) being connected to the gas outlet (11) of the plasma applicator (1), and/or
d) a gas source (12) being connected to the gas inlet (10) of the plasma applicator (1), and/or
e) an inlet valve (14) being arranged between the gas source (12) and the gas inlet (10) of the plasma applicator (1), and/or
f) an outlet valve (16) being arranged between the gas outlet (16) of the plasma applicator (1) and the suction pump (15), and/or
g) a control unit (20) for controlling the inlet valve (14), the outlet valve (16) and/or the high-voltage generator (18), and/or
h) a pressure sensor (21) measuring the pressure in the cavity of the plasma applicator (1), wherein the pressure sensor (21) is connected to the control unit (20) so that the control unit (20) controls the inlet valve (14), the outlet valve (16) and/or the high-voltage generator (18) depending on the measured pressure.

12. Use of a plasma applicator (1) according to any of the preceding claims for
a) the treatment of wounds (2), or
b) the treatment of living tissue, or
c) the treatment of skin diseases or skin disorders,

13. Method for applying a non-thermal plasma to a locally bounded surface, particularly for the treatment of wounds (2), **characterized by** the following steps:
a) Attaching a sealing cover (4) to the locally bounded surface thereby providing a cavity between the sealing cover (4) and the locally bounded surface,
b) Providing a non-thermal plasma within the cavity in contact with the locally bounded surface.

14. Method according to claim 13, further comprising the following steps:
a) Sealing the cavity substantially gastight, and/or
b) Reducing the pressure within the cavity by exhausting gas out of the cavity, and/or
c) Introducing a carrier gas into the cavity, and/or
d) Exciting an electrode arrangement in the cavity thereby generating the non-thermal plasma in the cavity.

15. Method according to any of claims 13 or 14, further comprising the following steps for energizing the electrode arrangement, which comprises a single electrode (8):
a) Electrically grounding the treated surface,
b) Exciting the electrode (8).

16. Method according to any of claims 13 to 15, wherein the locally bounded surface is a wound in the skin of a human or animal body.
